**Europäisches Patentamt**

**European Patent Office**

**Office européen des brevets**

(19)

(11) Veröffentlichungsnummer: **0 101 880**
**B1**

(12) # EUROPÄISCHE PATENTSCHRIFT

(45) Veröffentlichungstag der Patentschrift:
**08.02.89**

(51) Int. Cl.⁴: **G 01 N 33/48,** G 01 N 27/46,
G 01 N 27/02

(21) Anmeldenummer: **83107037.0**

(22) Anmeldetag: **18.07.83**

(54) Verfahren zur Bestimmung der Konzentration elektrochemisch umsetzbarer Stoffe.

(30) Priorität: 30.07.82 DE 3228542

(43) Veröffentlichungstag der Anmeldung:
07.03.84 Patentblatt 84/10

(45) Bekanntmachung des Hinweises auf die Patenterteilung:
08.02.89 Patentblatt 89/6

(84) Benannte Vertragsstaaten:
AT DE FR GB IT NL SE

(56) Entgegenhaltungen:
FR-A- 2 351 412
FR-A- 2 406 825
GB-A- 1 539 393

SIEMENS FORSCHUNGS- UND
ENTWICKLUNGSBERICHTE, Band 12, Nr. 2, April 1983,
Seiten 91-95, Würzburg, DE; U. GEBHARDT et al.:
"Electrocatalytic glucose sensor"

(73) Patentinhaber: Siemens Aktiengesellschaft Berlin und
München, Wittelsbacherplatz 2, D-8000 München 2 (DE)

(72) Erfinder: Preidel, Walter, Dr., Eskilstunastrasse 14,
D-8520 Erlangen (DE)
Erfinder: Mund, Konrad, Dr., Langenbrucker Weg 10,
D-8521 Uttenreuth (DE)

## Beschreibung

Die Erfindung betrifft ein Verfahren zur Bestimmung der Konzentration von elektrochemisch umsetzbaren Stoffen in einer Lösung mittels einer eine Messelektrode aufweisenden Messzelle.

Aus der FR-A-2 351 412 ist ein Verfahren zur differentiellen Impulspolarographie bekannt, bei dem der Messelektrode ein Potential aufgeprägt wird, das sich aus dem Grundpotential (zur Depolarisierung der Elektrode vor jeder Messung) und einer Reihe von Impulsen zusammensetzt, von denen jeder die Summe eines Hauptimpulses und eines Nebenimpulses geringer Amplitude ist, wobei sich der Nebenimpuls dem Hauptimpuls während des letzten Teils des Hauptimpulses überlagert und genau mit diesem zuendegeht. Haupt- und Nebenimpuls sind dabei rechteckförmig, die Amplitude des Hauptimpulses kann in geeigneter Weise zeitveränderlich sein.

Das bekannte Verfahren dient zur Konzentrationsbestimmung chemischer Spezies in wässrigen oder nicht-wässrigen Lösungen, insbesondere zur quantitativen Bestimmung gelöster Metallionen.

Die Bestimmung der Konzentration gelöster Stoffe, insbesondere in Gegenwart störender Fremdsubstanzen, ist auf vielen Gebieten von Bedeutung. Beispielhaft sei hierzu die Bestimmung von Glucose in Körperflüssigkeiten, wie Blut, genannt. Eine derartige Bestimmung ist insbesondere bei Diabetikern erforderlich.

Die Diabetestherapie kann wesentlich verbessert werden, wenn die Patienten fortwährend, d.h. bei einer konstanten Basalrate, mit Insulin versorgt werden und wenn vor den Mahlzeiten zusätzliche Insulindosen abgerufen werden. Für dieses Prinzip sind bereits implantierbare Pumpen entwickelt worden, die mit einem insulingefüllten Vorratsbehälter verbunden sind. Die Pumpen werden dabei durch eine programmierbare Steuereinheit geführt. Angestrebt wird jedoch ein geschlossener Regelkreis, wozu aber ein Sensor benötigt wird, der die Konzentration der Glucose im Blut des Patienten ermittelt.

Zur Glucosebestimmung sind bereits implantierbare elektrokatalytische Zuckersensoren bekannt. Derartige Sensoren weisen eine Messelektrode, beispielsweise aus Platin, auf, vor der eine diffusionsbestimmende Membran angeordnet sein kann (siehe dazu: DE-A-2 817 363). Zur Glucosebestimmung wird der Messelektrode dabei potentiostatisch ein Potentialprofil aufgeprägt, wobei zwei Niveaus vorgegeben werden: Ein positives und ein negatives Potential. Beim positiven Potential wird die Messelektrode reaktiviert, d.h. die auf der Elektrode befindlichen Reaktionsprodukte werden oxidiert. Wenn die Messelektrode das negative Potential, d.h. das Messpotential, erreicht, wird der Strom integriert; die dabei ermittelte Ladung ist das Messsignal, das der Glucosekonzentration zugeordnet wird. Bei den implantierbaren elektrokatalytischen Glucosesensoren bereitet es jedoch Schwierigkeiten, den Einfluss von Störfaktoren, wie Harnstoff und Aminosäuren, die Bestandteil der Körperflüssigkeit sind, zu unterdrücken.

Aufgabe der Erfindung ist es, ein Verfahren zur Bestimmung der Konzentration von elektrochemisch umsetzbaren Stoffen in einer Lösung mittels einer eine Messelektrode aufweisenden Messzelle in der Weise auszugestalten, dass eine empfindliche und über lange Zeit zuverlässige Bestimmung der Konzentration, auch in Gegenwart anderer Reaktanten, möglich ist.

Dies wird beim Verfahren gemäss der Erfindung dadurch erreicht, dass der Messelektrode ein innerhalb eines Potentialintervalls zeitlich in Potentialstufen variierendes Potential aufgeprägt wird, dass diesem Potential eine Wechselspannung vorgegebener Amplitude und Frequenz überlagert wird, und dass wenigstens für eine Potentialstufe der Real- und/oder der Imaginäranteil der Impedanz der Messelektrode ermittelt und daraus die Konzentration bestimmt wird.

Vorzugsweise wird beim erfindungsgemässen Verfahren der Realanteil und der Imaginäranteil der Impedanz ermittelt und zwar bei mehreren Potentialstufen.

Beim erfindungsgemässen Verfahren wird, um ein hohes Mass an Informationen zu erhalten, der Gleichspannung eine Wechselspannung überlagert und quasi die Antwort des angesprochenen elektrochemischen Systems analysiert. Als Messgrösse erhält man die Impedanz in Form von deren Real- und Imaginäranteil, und zwar in Abhängigkeit von der Wechselspannungsfrequenz und vom Potential. Somit ist es möglich, ein potentialabhängiges Impedanzspektrum der elektrochemischen Reaktion zu messen und die elektrischen Grössen beispielsweise als Ersatzschaltbild für diese Reaktion zu beschreiben.

Das erfindungsgemässe Verfahren eignet sich insbesondere zur Konzentrationsbestimmung von Glucose, Harnstoff und Aminosäuren in Flüssigkeiten, insbesondere Körperflüssigkeiten; bei den Aminosäuren wird dabei die Gesamtkonzentration ermittelt. Bei der Untersuchung von physiologischen Lösungen ist es auch möglich, die vorstehend genannten Stoffe nebeneinander, d.h. gleichzeitig zu bestimmen. Ferner eignet sich das erfindungsgemässe Verfahren auch zur Bestimmung der Konzentration von in Flüssigkeiten gelösten Gasen, insbesondere Sauerstoff und Chlor.

Beim erfindungsgemässen Verfahren wird der Messelektrode das Potential im allgemeinen mittels eines Potentiostaten aufgeprägt. Das Potential pendelt dabei zwischen zwei Grenzen, die sich nach den untersuchten Stoffen richten. So liegt beispielsweise bei der Bestimmung von Glucose, von Harnstoff und von Aminosäuren der Potentialbereich zwischen dem Potential der reversiblen Wasserstoffelektrode ($H_{2rev}$) und 1650 mV, bei der Bestimmung von Sauerstoff oder von Chlor wird folgender Potentialbereich gewählt:

$$250 \text{ mV} \leq H_{2rev} \leq 1450 \text{ mV}.$$

Allgemein richtet sich das jeweilige Potentialintervall nach der zu bestimmenden Substanz, nach den Begleitsubstanzen, nach der Art des Elektrodenmaterials und gegebenenfalls nach der vor der Messelektrode befindlichen Membran.

Das Potential, d.h. das Grundprofil des Potentials, besteht beim erfindungsgemässen Verfahren aus einer Anzahl von Stufen; diese Potentialstufen können voneinander den gleichen oder einen unterschiedlichen Abstand haben. Dem Grundprofil wird dann eine Wechselspannung vorgegebener Amplitude und Frequenz überlagert. Die Amplitude soll dabei klein sein im Vergleich zur Intervallbreite des Potentials; sie beträgt vorzugsweise 10 mV, kann beispielsweise aber auch Werte bis zu 100 mV annehmen. Die höchste nutzbare Frequenz liegt bei 10 kHz und ist durch die Doppelschichtkapazität der Elektrode sowie gegebenenfalls durch den Membranwiderstand vorgegeben. Die untere Grenze der Frequenz wird durch die zulässige Zyklendauer bedingt.

Beim erfindungsgemässen Verfahren wird für die einzelnen Potentialstufen der Real- und/oder der Imaginäranteil der Elektrodenimpedanz ermittelt. Beide Anteile werden dabei durch die Konzentration der in der Lösung vorhandenen Stoffe beeinflusst; zusätzlich ergibt sich auch noch ein Einfluss des Potentials. Wird nun eine Eichung durchgeführt und die Impedanz als Funktion der verschiedenen Potentiale bei fester Amplitude und Frequenz bestimmt, so erhält man die Koeffizienten eines Auswertungsschemas, anhand dessen die Konzentration der zu bestimmenden Stoffe ermittelt werden kann.

Wesentlich ist beim erfindungsgemässen Verfahren, dass an die katalytische Aktivität der Messelektrode keine erhöhten Anforderungen zu stellen sind. Voraussetzung ist allerdings, dass die Reaktanten an der Elektrode umgesetzt werden. Vorzugsweise wird bei diesem Verfahren eine Platinelektrode verwendet. Von Vorteil ist es beim erfindungsgemässen Verfahren ferner, wenn vor der Messelektrode eine Membran angeordnet wird. Diese Membran hat dann die Aufgabe, insbesondere grosse Moleküle, wie Eiweissstoffe, die stören können, von der Elektrode fernzuhalten und somit eine Umsetzung zu verhindern. Die Membran kann aber auch als Diffusionsbegrenzung für die durch sie hindurchdiffundierenden Stoffe dienen und auf diese Weise eine Vorselektierung von kleineren Molekülen bewirken.

Die der Messelektrode vorgelagerte Membran, die einen hydrophilen Charakter besitzt, weist vorzugsweise eine Dicke $d < 50 \ \mu m$ auf. Darüber hinaus soll diese Membran einen möglichst kleinen Diffusionskoeffizienten besitzen; angestrebt wird ein Diffusionskoeffizient $D < 10^{-8} \ cm^2 \cdot s^{-1}$. Zur Herstellung derartiger Membranen kann von Kunststoffen ausgegangen werden, die relativ hydrophobe Folien bilden; diese Kunststoffe werden dann durch geeignete Massnahmen hydrophiliert.

Das erfindungsgemässe Verfahren ermöglicht die analytische Erfassung der an der Messelektrode elektrochemisch reagierenden oder adsorbierten Stoffe. Mit diesem Verfahren können auch mehrere elektrochemisch wirksame Einzelkomponenten nebeneinander bestimmt werden. So ist es beispielsweise möglich, die Konzentration von Glucose auch in Anwesenheit von Harnstoff und Aminosäuren zu ermitteln.

Wird beispielsweise bei der Bestimmung der Glucosekonzentration ($v = 1$ Hz) lediglich die Kapazität der Messelektrode für die Auswertung herangezogen, so ergibt sich für die Konzentration ein Messfehler von etwa 20%, wenn das Potentialintervall in elf äquidistante Potentialstufen eingeteilt wird und diese Potentialstufen gleichrangig ausgewertet werden. Werden für die Auswertung dagegen diejenigen Potentialstufen ausgewählt, die bei Variation der Konzentration die ausgeprägtesten Änderungen der Impedanz zeigen und werden sowohl der Real- als auch der Imaginäranteil der Impedanz berücksichtigt, so kann der Messfehler auf unter 10% gesenkt werden.

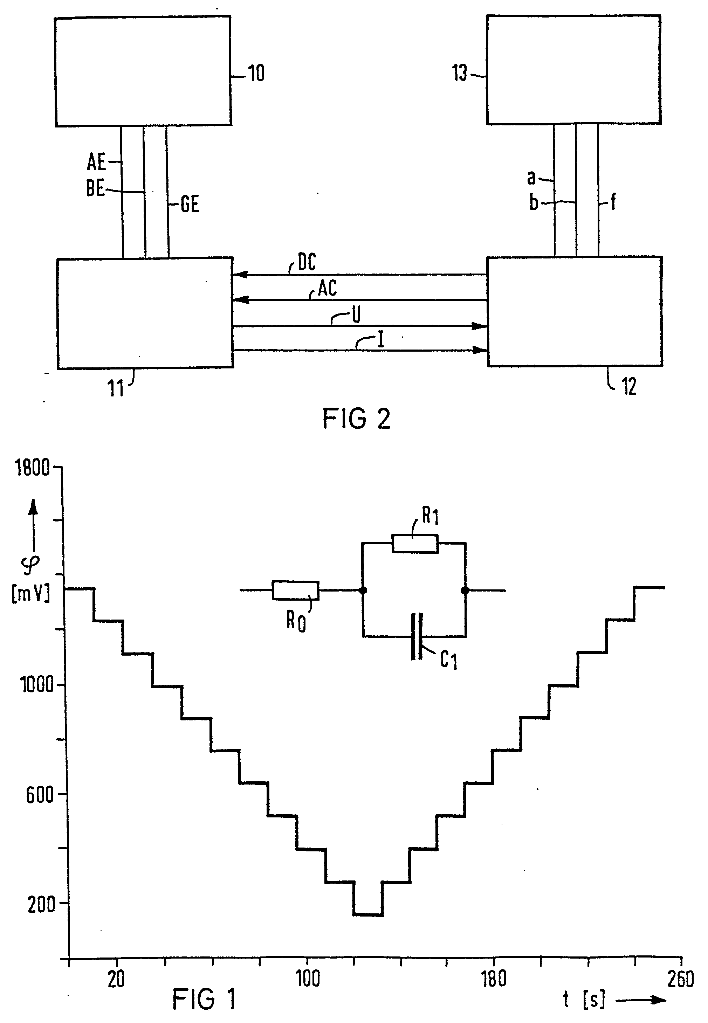

Anhand von Ausführungsbeispielen und Figuren soll die Erfindung noch näher erläutert werden. In Fig. 1 ist das Ersatzschaltbild einer Anordnung aus Messelektrode und Membran sowie der zeitliche Verlauf des Potentials dargestellt; Fig. 2 zeigt das Blockschaltbild einer Vorrichtung zur Durchführung des erfindungsgemässen Verfahrens.

Bei der Konzentrationsbestimmung entsprechend dem erfindungsgemässen Verfahren gelangt ein elektrochemischer Sensor zum Einsatz, welcher eine Messzelle, die beispielsweise aus Polymethylmethacrylat besteht, aufweist. Innerhalb der Messzelle befindet sich eine Mess- oder Arbeitselektrode (AE) aus Platin (aktive Fläche: beispielsweise ca. 0,1 cm²), vor der gegebenenfalls eine Membran angeordnet ist. Die Membran besteht beispielsweise aus Polytetrafluoräthylen, das mit quaternisiertem Benzylamin gegraftet ist; die Membran kann beispielsweise aber auch aus sulfoniertem Polysulfon bestehen. Auf der anderen Seite der Membran ist eine Gegenelektrode (GE), beispielsweise in Form eines platinierten Platinbleches, angeordnet. Die Messzelle, durch welche die die zu bestimmenden Stoffe enthaltende Lösung in einem geschlossenen Kreislauf geführt wird, ist schliesslich noch mit einer Bezugselektrode (BE), beispielsweise in Form einer Hg/Hg$_2$Cl$_2$-Elektrode, verbunden. Die Bezugselektrode kann, bezogen auf die Membran, auf der Seite der Gegenelektrode oder auf der Seite der Messelektrode angeordnet sein. Im letzten Fall wird verhindert, dass die Bezugselektrode durch Verunreinigungen der Lösung oder durch Bestandteile der Körperflüssigkeit geschädigt wird. Dies kann vorteilhaft auch dadurch vermieden werden, dass eine hochkapazitive Gegenelektrode verwendet wird, die gleichzeitig als Bezugselektrode dient.

Beim Impedanzverfahren, wie das erfindungsgemässe Verfahren kurz auch bezeichnet wird, ist es für die Auswertung günstig, wenn der elektrochemischen Messzelle, d.h. der Anordnung aus Messelektrode und Membran, ein Ersatzschaltbild zugeordnet wird und die elektrischen Grössen dieses Ersatzschaltbildes dann messtechnisch erfasst werden. Das Ersatzschaltbild weist einen

Widerstand $R_1$ und einen parallel dazu geschalteten Kondensator mit der Kapazität $C_1$ auf; diese Anordnung aus Widerstand und Kondensator ist mit einem weiteren Widerstand $R_0$ in Reihe geschaltet (siehe Fig. 1). Das Ersatzschaltbild ändert sich in Abhängigkeit vom Potential an der Messelektrode und in Abhängigkeit von der Konzentration, beispielsweise der Glucosekonzentration; dabei ändern sich jedoch nur die Werte der elektrischen Grössen, nicht aber deren Anordnung.

Der Aufbau des Ersatzschaltbildes kann unmittelbar der Art und dem Verlauf der sogenannten Ortskurve entnommen werden. Die Ortskurve (in Gestalt eines Halbkreises) ist eine Darstellung des Imaginäranteils der Impedanz gegen den Realanteil; unter Impedanz wird im übrigen der Scheinwiderstand in der Wechselstromtechnik verstanden. Der Zahlenwert der Impedanz ergibt sich aus dem Quotienten $U_{eff}/I_{eff}$. Bei einer rein sinusförmigen Spannung gilt:

$$U = U_0 \cdot \sin(\omega t + \varphi) \text{ und } U_{eff} = U_0 \cdot \sqrt{2/2},$$

mit $\omega$ = Kreisfrequenz ($2\pi\nu$), $\varphi$ = Phasenverschiebung (zwischen Spannung und Strom) und $U_0$ = Amplitude; entsprechendes gilt für den Strom I.

Der Realanteil der Impedanz ist definiert als

$$a \frac{U_{eff}}{I_{eff}} \cdot \cos(\varphi),$$

der Imaginäranteil ist $b = \frac{U_{eff}}{I_{eff}} \cdot \sin(\varphi)$.

Wird nun die Impedanz als Funktion der Frequenz $\nu$ aufgenommen und dann in Form der Ortskurve dargestellt, so erhält man aus dem Kurvenverlauf Aufschluss über die Anordnung der elektrischen Grössen (Widerstände und Kondensatoren) und aus den Zahlenwerten die elektrischen Werte der Elemente des Ersatzschaltbildes. Für das vorstehend beschriebene Ersatzschaltbild gilt dabei folgendes:

Bei hoher Frequenz: $R_0 = a$;
bei niedriger Frequenz: $R_1 = a$;
bei mittlerer Frequenz: $C_1 = \dfrac{1}{\omega \cdot b}$.

Wird die Impedanz in Abhängigkeit von der Frequenz für jede Potentialstufe einer – sich bei der Überlagerung von Dreieckspannungskurven mit Wechselstrom ergebenden – Potentialtreppe (siehe Fig. 1) zyklisch gemessen, so werden für die einzelnen Stufen die jeweiligen Widerstands- und Kapazitätswerte der elektrischen Grössen des Ersatzschaltbildes erhalten. Eine Messung der Impedanz für viele Frequenzen erübrigt sich dabei dann, wenn zwei Einzelfrequenzen in der Ortskurve günstig ausgewählt werden. In geometrischer Hinsicht bedeutet dies: Der Halbkreis ist dann gut zu konstruieren, wenn zwei genügend weit voneinander entfernte Punkte (des Kreisumfangs) bekannt sind.

Trägt man die Kapazität bzw. den Widerstand der einzelnen Potentialstufen gegen die Konzentration auf, so erhält man für bestimmte Potentialstufen eine Gerade; dies bedeutet, dass die Konzentration linear abhängig ist von der elektrischen Grösse. Sind verschiedene Stoffe in der untersuchten Lösung vorhanden und machen sich diese Substanzen, was im allgemeinen der Fall ist, bei unterschiedlichen Potentialen bemerkbar, so ist auch eine Bestimmung sämtlicher Stoffe nebeneinander möglich.

Zur Bestimmung der elektrischen Grössen der Messelektrode (mit vorgelagerter Membran) dient beispielsweise ein Potentiostat. An den Eingang des Potentiostaten 11 werden, wie aus Fig. 2 ersichtlich, die sich zeitlich ändernde Gleichspannung (DC) und die zu überlagernde sinusförmige Wechselspannung (AC) gegeben. Diese beiden Spannungen können in der Gleichspannungsquelle bzw. im Oszillator eines Frequenzganganalysators 12 erzeugt werden. Die Antwort des elektrochemischen Systems, d.h. der Messzelle 10, auf die Wechselspannung wird als Spannung zwischen Messelektrode (AE) und Bezugselektrode (BE) vom y-Eingang abgenommen. Der Abgriff am Widerstand im Gegenelektrodenkreis (GE) dient zur Information über den Strom, gemessen über den x-Eingang. Aus den Eingangsgrössen I und U werden im Frequenzganganalysator die Effektivwerte der Spannung für die am Oszillator eingestellte Frequenz (sogenanntes Lock-in-Prinzip) sowie der jeweilige Phasenwinkel zur Oszillatorphase ermittelt. Aus den Werten für den Realanteil (a) und den Imaginäranteil (b) der Impedanz sowie für die Frequenz (f) wird dann, beispielsweise mittels eines Rechners 13, die Konzentration bestimmt.

Unter Verwendung einer Raney-Platin-Elektrode als Messelektrode wurde in einer Reihe von Versuchen (1) die Konzentration von Glucose in Tyrodelösung (eine mit Blut isotonische Lösung) in Gegenwart von Harnstoff bestimmt. Die Impedanz der Messelektrode, der keine Membran vorgelagert war, wurde bei Frequenzen von 100 Hz bis 0,1 Hz für jeweils eine Frequenz bei jeder Potentialstufe gemessen. Der Potentialbereich lag zwischen 0 und 1650 mV, gemessen gegen die reversible Wasserstoffelektrode; jede Potentialstufe betrug 30 mV (Dauer einer Stufe: 10 bis 60 s). Bei diesen Messungen zeigte sich, dass eine Glucosebestimmung möglich ist, auch in Gegenwart von Harnstoff im normalen physiologischen Konzentrationsbereich. Entsprechendes gilt für die Anwesenheit physiologischer Mengen von Aminosäuren.

Bei den vorstehend beschriebenen Versuchen (1) betrug die Messzeit etwa 30 min. Eine Reduzierung der Messzeit auf ca. 6 min gelingt unter folgenden Bedingungen:

– Vergrösserung der Potentialstufen auf 150 mV (bei einer Dauer von ca. 20 s);
– Frequenz 1 Hz;
– Amplitude 14 mV;
– Verwendung einer Membran vor der Platinelektrode (die Membran bestand aus gegraftetem Polytetrafluoräthylen und wies eine Dicke von 25 μm auf).

Auch bei diesen Versuchen (2) ergab sich, dass Glucose in Gegenwart wechselnder Mengen an Harnstoff und Aminosäuren (im physiologischen Konzentrationsbereich) zuverlässig bestimmt werden kann; die Messungen erfolgten im Potentialbereich von 0 bis 1650 mV. Die Aminosäuren gelangten dabei jeweils als Gemisch sämtlicher physiologischer Verbindungen zur Anwendung.

Bei den vorstehend beschriebenen Versuchen (2) war der Elektrolyt, d.h. die Tyrodelösung, mit einem $N_2/CO_2$-Gemisch (95:5) gesättigt worden. Wird dieses Gemisch durch ein Pressluft/$CO_2$-Gemisch ersetzt, d.h. durch ein Gemisch aus $O_2$, $N_2$ und $CO_2$, so zeigt sich, dass sich durch den Sauerstoffeinfluss Veränderungen im Potentialverlauf der a- und b-Werte ergeben. Es ist dadurch aber andererseits auch möglich, den Sauerstoffgehalt zu bestimmen.

Bei bestimmten Überwachungssystemen, beispielsweise in Vorrichtungen zur Eliminierung von Harnstoff (vgl.: DE-OS 3 040 470), ist es erforderlich, den Sauerstoff- und/oder Chlorgehalt zu ermitteln. Auch dies ist mit Hilfe des erfindungsgemässen Verfahrens möglich. Dazu werden Real- und Imaginäranteil der Impedanz von zwei Frequenzen an einer platinierten Platinelektrode bei einer mit Wechselstrom überlagerten Potentialtreppe unter folgenden Bedingungen gemessen (die Messungen erfolgen ohne die Verwendung einer Membran):

Potential: $250\,mV \leq H_{2rev} \leq 1450\,mV$;
Potentialstufe: 120 mV (Dauer: 12 s);
Amplitude: 14 mV;
Frequenz: 3 Hz und 1 kHz (jeweils bei jeder Potentialstufe).

Durch Auswertung der a- und b-Werte erhält man die Sauerstoff- bzw. Chlorkonzentration; Messdauer: 5 min. Dabei zeigte sich, dass der maximale Fehler bei der Sauerstoffbestimmung 0,1 mg/dl beträgt, und zwar im Bereich von 0 bis 4,1 mg/dl, und derjenige bei der Chlorbestimmung 2 mg/dl, im Bereich von 0 bis 23 mg/dl (Elektrolyt: gepufferte 1 n KCl-Lösung).

Das erfindungsgemässe Verfahren bietet somit die Möglichkeit, die Konzentration von elektrochemisch an einer Elektrode umsetzbaren Stoffen zu bestimmen. Dabei kann die Bestimmung – aufgrund der vielen Variationsmöglichkeiten – gut an das jeweilige System angepasst werden. So ist eine Änderung der Spannungsgeschwindigkeit ebenso möglich wie eine (zusätzliche) Änderung der Messfrequenz. Ferner kann die Impedanz bei einem Potential kurzzeitig nacheinander mit verschiedenen Frequenzen gemessen werden. Es besteht dann nämlich die Möglichkeit, bei geeigneter Wahl der Frequenzen den Membranwiderstand und den Durchtrittswiderstand voneinander zu trennen. Auf diese Weise könnte bei der Auswertung eine im Lauf der Zeit erfolgende Veränderung des ohmschen Widerstandes der Membran berücksichtigt werden. Die Lage des Potentialintervalls richtet sich beim erfindungsgemässen Verfahren im übrigen nach dem untersuchten System, und sie kann durch zyklische voltametrische Untersuchungen leicht bestimmt werden. Die Höhe der Potentialstufen wird sich im allgemeinen nach dem Abstand der einzelnen Potentiale voneinander und nach der für einen vollen Messzyklus angestrebten Zeit richten.

## Patentansprüche

1. Verfahren zur Bestimmung der Konzentration von elektrochemisch umsetzbaren Stoffen in einer Lösung mittels einer eine Messelektrode aufweisenden Messzelle, wobei der Messelektrode ein innerhalb eines Potentialintervalls zeitlich in Potentialstufen variierendes Potential aufgeprägt wird, und wobei diesem Potential eine Wechselspannung vorgegebener Amplitude und Frequenz überlagert wird, und wobei wenigstens für eine Potentialstufe der Real- und/oder der Imaginäranteil der Impedanz der Messelektrode ermittelt und daraus die Konzentration bestimmt wird.

2. Verfahren nach Anspruch 1, wobei bei mehreren Potentialstufen der Real- und der Imaginäranteil der Impedanz ermittelt wird.

3. Verfahren nach Anspruch 1 oder 2, wobei das Potential bei wenigstens einer Potentialstufe mit Wechselspannung verschiedener Frequenzen überlagert wird.

4. Verfahren nach einem der Ansprüche 1 bis 3, wobei eine Messelektrode aus Platin verwendet wird.

5. Verfahren nach einem der Ansprüche 1 bis 4, wobei vor der Messelektrode eine Membran angeordnet wird.

## Claims

1. A method of determining the concentration of electrochemically transformable substances in a solution by means of a measuring cell having a measuring electrode, wherein the measuring electrode is impressed with a potential varying with time in potential steps within a potential interval and wherein this potential has superposed on it an alternating voltage of predetermined amplitude and frequency, and wherein at least for one potential step the real and/or the imaginary component of the impedance of the measuring electrode is determined and the concentration is determined therefrom.

2. A method according to claim 1, wherein the real and the imaginary component of the impedance is calculated in a plurality of potential steps.

3. A method according to claim 1 or claim 2, wherein in at least one potential step the potential is superposed with alternating voltage having different frequencies.

4. A method according to any one of claims 1 to 3, wherein a measuring electrode of platinum is used.

5. A method according to any one of claims 1 to 4, wherein a membrane is arranged in front of the measuring electrode.

## Revendications

1. Procédé de détermination de la concentration en solution de substances réagissant électrochi- miquement, au moyen d'une cellule de mesure comprenant une électrode de mesure, qui consiste à appliquer à l'électrode de mesure un potentiel variant par paliers en fonction du temps, dans un intervalle de potentiel, et à superposer à ce potentiel une tension alternative d'amplitude et de fréquence prescrite et à déterminer, au moins pour un palier de potentiel, la partie réelle et/ou la partie imaginaire de l'impédance de l'électrode de mesure, et à en déduire la concentration.

2. Procédé suivant la revendication 1, qui consiste à déterminer la partie réelle et la partie imaginaire de l'impédance pour plusieurs paliers de potentiel.

3. Procédé suivant la revendication 1 ou 2, qui consiste à superposer au potentiel une tension alternative de fréquence différente pour au moins un palier de potentiel.

4. Procédé suivant l'une des revendications 1 à 3, qui consiste à utiliser une électrode de mesure en platine.

5. Procédé suivant l'une des revendications 1 à 4, qui consiste à disposer une membrane devant l'électrode de mesure.

10

AE
BE
GE

13

a
b
f

DC
AC
U
I

11

12

## FIG 2

φ
[mV]

1800

1000

600

200

R1

R0

C1

20          100          180          260

## FIG 1

t [s]